# EUROPÄISCHE PATENTANMELDUNG

(11) **EP 4 809 948 A2**
(43) Veröffentlichungstag der Anmeldung: **23.09.2026**
(21) Anmeldenummer: 26197807.6
(22) Anmeldetag: 17.07.2020
(51) Int. Cl.: A61B 17/64

(54) **ÄUSSERE HALTEVORRICHUNG FÜR KNOCHENBRÜCHE**

(62) Dokumentnummer(n) der früheren Anmeldung(en) nach Art. 76 EPÜ: 20750581.9 / 4 181 804
(71) Anmelder: AO Technology AG, 7000 Chur (CH)
(72) Erfinder: Höntzsch, Dankward, 72076 Tubingen (DE); Stehr, Werner, 72160 Horb (DE)
(74) Vertreter: Witte, Weller & Partner Patentanwälte mbB

(57) **Zusammenfassung**

Die Erfindung schlägt vor, ein Gelenk (4) einer äußeren Haltevorrichtung (1) für Bruchstücke eines Knochens (Fixateur externe) mit kreisförmigen Rippen (19, 29) und Nuten (22) mit V-förmigem Querschnitt zu versehen, deren Flanken kegelstumpfförmige Reibflächen (20, 21, 23, 24, 25, 26, 30, 31) bilden, die eine hohe Reibkraft gegen Verdrehen des Gelenks (4) und damit gegen ein ungewolltes verstellen der Haltevorrichtung (1) und ein Verstellen der Haltevorrichtung (1) mit einstellbarer Reibkraft zum Ausrichten der Bruchstücke des Knochens ermöglichen.

## Beschreibung

Die Erfindung betrifft eine äußere Haltevorrichtung zum Halten von Bruchstücken eines Knochens nach einem Knochenbruch oder allgemein von Skelettteilen mit den Merkmalen des Oberbegriffs des Anspruchs 1.

Solche Haltevorrichtungen werden auch als Fixateur externe bezeichnet, wobei es unterschiedlichste Ausführungen gibt. Gemeinsam ist diesen Haltevorrichtungen, dass sie es ermöglichen, zwei oder auch mehr Bruchstücke eines Knochens oder einzelner Skelettteile in einer gewünschten Lage zueinander aneinander zu befestigen. Die Bruchstücke werden in ihrer ursprünglichen Lage des nicht gebrochenen Knochens aneinander befestigt, damit sie in gewünschter Weise gehalten werden. Dazu werden Schrauben, Stifte, Drähte oder sonstige Befestigungselemente, die nachfolgend zusammenfassend als Pins bezeichnet werden, von außen durch die Haut an oder in den Bruchstücken des Knochens befestigt und die Pins ihrerseits an der Haltevorrichtung befestigt, wobei die Pins Bestandteile der Haltevorrichtung sein können, allerdings nicht sein müssen.

Das Gebrauchsmuster DE 84 19 372 U1 offenbart eine solche Haltevorrichtung mit Stäben und Gelenken, die die Stäbe verbinden. Dabei sind bei gelösten Gelenken die Stäbe um Achsen der Gelenke gegeneinander drehbar und durch Feststellen der Gelenke starr miteinander verbindbar. Die Gelenke weisen zwei U-förmige Klemmbacken mit einem an einem Ende offenen Schlitz, der mit einem Rundloch endet, durch das die Stäbe steckbar sind, auf. An den Stäben lassen sich - ebenfalls mit Gelenken - Pins befestigen, die in Bruchstücken eines gebrochenen Knochens befestigt sind. Die Klemmbacken der Gelenke sind durch eine Schraube verbunden, die die Klemmbacken mit Abstand von dem Rundloch und nahe an einem offenen Ende des Schlitzes senkrecht zum Schlitz verbindet, so dass die Klemmbacken um die Schraube drehbar sind. Durch Festziehen der Schraube werden die die Stäbe in den Rundlöchern der Klemmbacken festgespannt und die Klemmbacken gegeneinander gespannt. Die Klemmbacken weisen die Schraube umschließende Stirnverzahnungen an einander zugewandten Seiten auf, die die Klemmbacken durch Formschluss gegen Drehen halten, wenn sie zusammen gespannt sind. Zum Drehen der Klemmbacken muss die Schraube so weit gelöst werden, dass die Stirnverzahnungen der Klemmbacken außer Eingriff voneinander gelangen. Außerdem lassen sich die Klemmbacken nur in von den Stirnverzahnungen vorgegebenen Winkelschritten verstellen.

Aufgabe der Erfindung ist, eine Haltevorrichtung der vorstehend erläuterten Art vorzuschlagen, die ein Ausrichten von Bruchstücken eines gebrochenen Knochens in eine gewünschte Lage erleichtert.

Diese Aufgabe wird erfindungsgemäß durch die Merkmale des Anspruchs 1 gelöst. Die erfindungsgemäße äußere Haltevorrichtung zum Halten von Bruchstücken eines gebrochenen Knochens, die auch als Fixateur externe bezeichnet werden kann, weist zwei Stäbe und ein Gelenk auf, das die Stäbe um eine Achse des Gelenks drehbar verbindet, wenn es gelöst ist, und das die Stäbe aneinander festlegt, wenn das Gelenk festgestellt ist. Das Gelenk weist zwei Stabhalter auf, an oder in denen die Stäbe befestigt beziehungsweise befestigbar sind und die bei gelöstem Gelenk um die Achse des Gelenks gegeneinander drehbar sind.

Erfindungsgemäß weisen die Stabhalter des Gelenks an einander zugewandten Seiten kegelstumpfförmige, zu der Achse des Gelenks konzentrische und gegengleiche Reibflächen auf, die mit einer Spanneinrichtung zusammen spannbar sind.

"Kegelstumpfförmig" bedeutet genau genommen Mantelflächen eines Kegelstumpfs, wobei eine Achse des Kegelstumpfs mit der Achse des Gelenks zusammen fällt. Die zusammengespannten Reibflächen erzeugen aufgrund einer Keilwirkung eine hohe Reibung, die die beiden zusammen gespannten Stabhalter des Gelenks gegen Drehen hält. Die Reibung ist von einer Spannkraft, mit der die beiden Reibflächen zusammen gespannt werden, von einem Kegelwinkel der kegelstumpfförmigen Reibflächen, einer Materialpaarung und einem Oberflächenzustand der Reibflächen (trocken, feucht, geschmiert, verschmutzt ...) abhängig. Der Kegelwinkel wird so spitz gewählt, dass eine hohe Reibung erreicht wird, die die Stäbe gegen Verdrehen um die Achse des Gelenks hält, wobei eine Selbsthemmung, also ein Klemmen der zusammengespannten Reibflächen aneinander auch ohne Spannkraft vermieden werden soll.

Die Spanneinrichtung kann beispielsweise eine Schraube aufweisen, die mit der Achse des Gelenks gleichachsige Durchstecklöcher in den Stabhaltern durchsetzt und die in ein Innengewinde in einer der Spanneinrichtungen einschraubbar oder auf die eine Mutter aufgeschraubt ist.

"Gegengleich" bedeutet zueinander passende Reibflächenpaare, von denen eine die Form eines Innenkegelstumpfs und die andere die Form eines Außenkegelstumpfs mit gleichem Kegelwinkel und gleichem Durchmesser aufweist.

An den Stäben sind - mit erfindungsgemäßen Gelenken oder in anderer Weise - Pins befestigbar, die an oder in den Bruchstücken des gebrochenen Knochens befestigt beziehungsweise befestigbar sind. Solche Pins sind Schrauben, Stifte, Drähte oder andere Befestigungselemente.

Die erfindungsgemäße Haltevorrichtung kann mehr als zwei Stäbe aufweisen, von denen jeweils zwei winkeleinstellbar mit einem Gelenk verbunden sind. Parallele Stäbe können auch mit mehr als einem Gelenk verbunden werden. Außerdem kann die Haltevorrichtung Pins oder dergleichen zur Befestigung an oder in den Bruchstücken des gebrochenen Knochens aufweisen, die jeweils mit einem Gelenk an einem der Stäbe der Haltevorrichtung befestigt sind. Denkbar ist auch eine Befestigung eines Pins mit mehreren Gelenken an mehreren Stäben.

Die Erfindung ermöglicht eine hohe Reibkraft gegen Verdrehen der Stabhalter des Gelenks mit einer verhältnismäßig kleinen axialen Spannkraft. Ein weiterer Vorteil ist eine stufenlose Winkeleinstellbarkeit der Stäbe. Bei einem nur teilweisen Lockern des Gelenks lassen sich die Stäbe durch Überwinden der Reibung zwischen den Stabhaltern von Hand gegeneinander verschwenken und dadurch die an den Stäben befestigten Bruchstücke des gebrochenen Knochens ausrichten, wobei die Reibkraft so groß ist, dass die Bruchstücke ihre Lage ohne äußere Krafteinwirkung beibehalten. Durch Einstellen der Spannkraft lässt sich die Reibkraft der Gelenke gegen Schwenken der Stäbe individuell auf ein gewünschtes Maß einstellen. Sind die Bruchstücke in die gewünschte Lage gebracht, wird das Gelenk festgespannt und dadurch die Stäbe mit den an ihnen befestigten Bruchstücken des gebrochenen Knochens in ihrer Lage zueinander festgelegt. Es können mehr als zwei und prinzipiell beliebig viele Stäbe mit einer entsprechenden Anzahl an Gelenken miteinander verbunden werden.

Eine Weiterbildung der Erfindung sieht vor, dass jeder Stabhalter zwei oder auch mehr zueinander und zu der Achse des Gelenks konzentrische, kegelstumpfförmige Reibflächen aufweisen, die einander umschließen. Vorzugsweise weist jeder Stabhalter eine innenkegelstumpfförmige Reibfläche und eine außenkegelstumpfförmige Reibfläche auf, die die innenkegelstumpfförmige Reibfläche konzentrisch umschließt oder die von der innenkegelstumpfförmigen Reibfläche konzentrisch umschlossen wird. Ein zweiter Stabhalter des Gelenks weist zu den beiden Reibflächen eines ersten Stabhalters des Gelenks gegengleiche, kegelstumpfförmige Reibflächen auf. Die Reibflächen sind an einander zugewandten Seiten der Stabhalter angeordnet, so dass sie mit der Spanneinrichtung zusammen spannbar sind. Es weist beispielsweise ein Stabhalter eine kreisförmige, zu der Achse des Gelenks konzentrische Rippe mit einem umgekehrten V-Querschnitt an einer dem anderen Stabhalter desselben Gelenks zugewandten Seite und der andere Stabhalter eine ebenfalls kreisförmige und zu der Achse des Gelenks konzentrische Nut mit V-Querschnitt auf. "Umgekehrt" bedeutet, dass die Spitze des V-Querschnitts der Rippe der Nut zugewandt ist. Die Rippe und die Nut weisen gleiche Durchmesser und gegengleiche Querschnitte auf. Seitenflächen der Rippe und Nutwangen bilden die kegelstumpfförmigen Reibflächen. Die Spitze des V-Querschnitts der Rippe ist vorzugsweise abgeflacht, damit sie nicht an einem Nutgrund, der ebenfalls abgeflacht sein kann, aufsitzt. Die Reibflächen eines Stabhalters können gleiche oder verschiedene Kegelwinkel aufweisen, zugeordnete gegengleichen Reibflächen der beiden Stabhalter eines Gelenks weisen gleiche Kegelwinkel und gleiche Durchmesser auf. Die mit dieser Ausführung der Erfindung erreichte Verdoppelung oder Vervielfachung der Reibflächen verdoppelt oder vervielfacht die Reibung zwischen den Stabhaltern des Gelenks bei gleicher axialer Spannkraft, so dass die zusammengespannten Stabhalter zuverlässig gegen Verdrehen gegeneinander gehalten sind.

Ein weiterer Vorteil der Vielzahl an Reibflächen ist eine niedrige Flächenpressung, die bei einem Lockern der Spanneinrichtung zum Verstellen des Gelenks ein Gleiten der Reibflächen aufeinander verbessert. Die Stabhalter und mit ihnen die Stäbe lassen sich dadurch ruckfrei gegeneinander drehen.

Eine bevorzugte Ausgestaltung der Erfindung sieht eine weitere, kegelstumpfförmige und zu der Achse des Gelenks konzentrische Reibfläche an einer dem ersten Stabhalter abgewandten Seite des zweiten Stabhalters des Gelenks vor. Diese Reibfläche wirkt zusammen mit einer zu der weiteren Reibfläche gegengleichen Reibfläche, die drehfest und axial beweglich mit dem ersten Stabhalter verbunden und auf der dem ersten Stabhalter abgewandten Seite des zweiten Stabhalters angeordnet und dessen Reibfläche zugewandt ist. Die Reibflächenpaare auf der dem ersten Stabhalter abgewandten Seite des zweiten Stabhalters werden vorzugsweise mit derselben Spanneinrichtung zusammen gespannt wie die Stabhalter zum Zusammenspannen der Reibflächenpaare an ihren einander zugewandten Seiten. Das Reibflächenpaare auf der dem ersten Stabhalter abgewandten Seite des zweiten Stabhalters verdoppelt ebenfalls die Reibkraft zwischen den Stabhaltern des Gelenks gegen Verdrehen. Auch diese Ausgestaltung der Erfindung kann mehrere, einander konzentrisch umschließende innenkegelstumpfförmige und außenkegelstumpfförmige Reibflächen aufweisen, was die Reibkraft gegen Verdrehen nochmals verdoppelt beziehungsweise vervielfacht.

Zu einem drehfesten Verbinden der zu der weiteren Reibfläche des zweiten Stabhalters gegengleichen Reibfläche mit dem ersten Stabhalter sieht eine Ausgestaltung eine mit dem ersten Stabhalter drehfeste und zu der Achse des Gelenks koaxiale Welle vor, die ein beispielsweise zu der Achse des Gelenks koaxiales Durchgangsloch im zweiten Stabhalter durchgreift und an der die zu der weiteren Reibfläche gegengleiche Reibfläche drehfest und axial beweglich geführt ist. Die Welle kann Teil der Spanneinrichtung sein und die axiale Spannkraft zum gegeneinander Spannen aller Reibflächen des Gelenks zwischen dem ersten Stabhalter und der zu der weiteren Reibfläche gegengleichen Reibfläche übertragen. Beispielsweise kann die Welle von einem Gewindebolzen gebildet sein, der starr mit dem ersten Stabhalter ist oder von einer Schraube, deren Schraubenkopf drehfest und zugfest mit dem ersten Stabhalter verbunden ist. Die mit dem ersten Stabhalter drehfeste Welle hält die zu der weiteren Reibfläche gegengleiche Reibfläche auch gegen ein Mitdrehen mit dem zweiten Stabhalter, wenn die durch das Gelenk verbundenen Stäbe gegeneinander verschwenkt werden, wodurch die Spannkraft und die Reibkraft nicht durch das Schwenken der Stabhalter mit den Stäben verändert wird.

Eine Ausgestaltung der Erfindung sieht eine Abdrückfeder vor, die die Reibflächen voneinander abdrückt, wenn die Spannkraft verringert oder gelöst wird. Die Abdrückfeder wirkt einer Selbsthemmung, also einem Klemmen der kegelstumpfförmigen Reibflächen aneinander, entgegen und bewirkt eine leichtgängige Winkelverstellbarkeit der Stabhalter mit den Stäben mit einstellbarer Reibkraft durch Verringerung der Spannkraft.

Zum Ausgleich von Herstellungstoleranzen sieht eine Ausgestaltung der Erfindung radialelastisch ausgebildete Reibflächen vor, wobei es genügt, wenn ein Teil der Reibflächen radialelastisch ist.

Eine Ausgestaltung der Erfindung sieht lösbare Klemmen zum Festklemmen der Stäbe und/oder der Pins vor. Die Stabhalter können beispielsweise Spannbackenpaare aufweisen, zwischen denen die Stäbe und/oder die Pins angeordnet und eingespannt werden. Die Spannbackenpaare können mit der Spanneinrichtung zusammen gespannt werden, die auch die Reibflächen zusammen spannt. Die Spannbackenpaare können auch in anderer Weise zusammen gespannt werden. Die Spannbackenpaare können beispielsweise einstückig elastisch miteinander verbunden oder zwei- beziehungsweise mehrstückig sein.

Eine Ausgestaltung der Erfindung sieht vor, dass eine, mehrere oder alle Klemmen Klemmeinrichtungen für Stäbe mit unterschiedlichen Stabquerschnitten aufweist. Beispielsweise können die Spannbackenpaare der Klemmen einander zugewandte Nuten mit verschiedenen Querschnitten aufweisen, in denen die Stäbe oder Pins anordenbar und festspannbar sind.

Sämtliche in der Beschreibung genannte und/oder der Zeichnung dargestellte Merkmale können einzeln für sich oder in jeder beliebigen Kombination bei Ausführungen der Erfindung verwirklicht sein. Ausführungen der Erfindung, die nicht alle, sondern nur einen Teil der Merkmale eines Anspruchs, auch des unabhängigen Anspruchs, aufweisen, sind möglich.

Die Erfindung wird nachfolgend anhand eines in der Zeichnung dargestellten Ausführungsbeispiels näher erläutert. Es zeigen:
- Figur 1: eine Verwendung einer erfindungsgemäßen äußeren Haltevorrichtung zum Halten von Bruchstücken eines gebrochenen Knochens (Fixateur externe);
- Figur 2: eine perspektivische Darstellung von zwei Stäben und einem die Stäbe verbindenden Gelenk der Haltevorrichtung aus Figur 1 in perspektivischer Darstellung;
- Figur 3: eine perspektivische Achsschnittdarstellung des Gelenks aus Figur 2;
- Figur 4: eine perspektivische Explosionsdarstellung von Einzelteilen des Gelenks aus Figur 3; und
- Figur 5: ein Einzelteil des Gelenks aus Figur 2 in einer abgewandelten Ausführungsform der Erfindung in perspektivischer Darstellung.

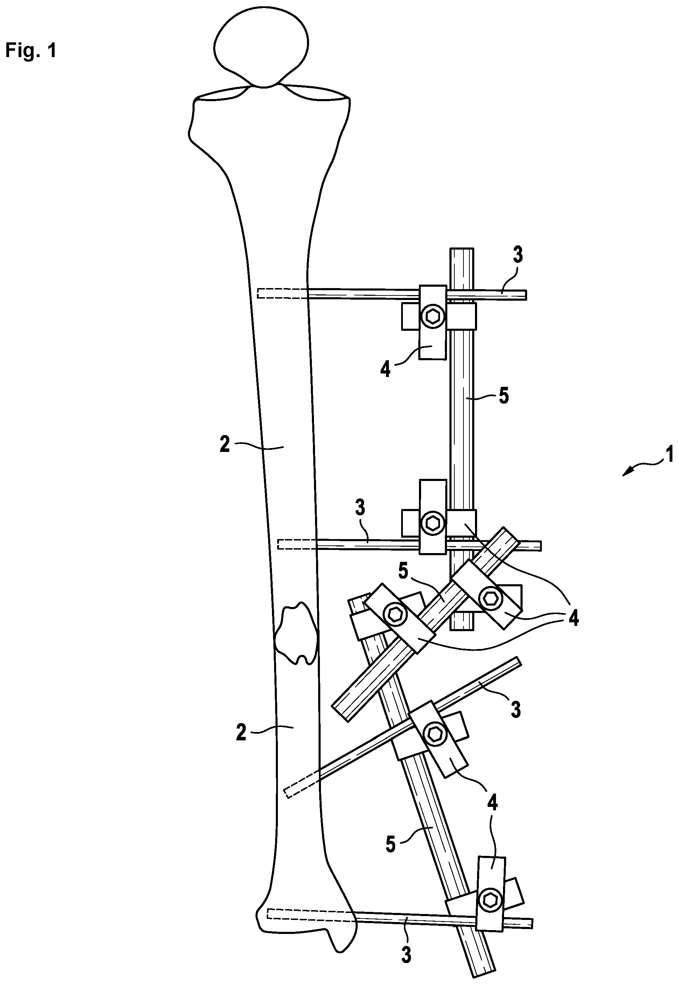

Die in Figur 1 dargestellte, erfindungsgemäße, äußere Haltevorrichtung 1 dient zu einem Verbinden von Bruchstücken 2 eines gebrochenen Knochens oder allgemein zum Halten von Skelettteilen in einer vorgesehenen Lage zueinander. Die äußere Haltevorrichtung 1 wird auch als Fixateur externe bezeichnet.

Zu einer Befestigung der Haltevorrichtung 1 an den Bruchstücken 2 des gebrochenen Knochens oder umgekehrt zur Befestigung der Bruchstücke 2 an der Haltevorrichtung 1 sind beziehungsweise werden in jedes Bruchstück 2 ein oder mehrere Knochenschrauben von außen durch die Haut eines Patienten geschraubt. Anstelle der Knochenschrauben können auch Stifte, Drähte oder andere, jetzt erhältliche oder zukünftig entwickelte, stift- oder andersförmige Befestigungselemente an oder in den Bruchstücken 2 des Knochens befestigt werden. Die Befestigungselemente werden hier als Pins 3 bezeichnet, unabhängig davon, ob sie die Form eines Pins haben.

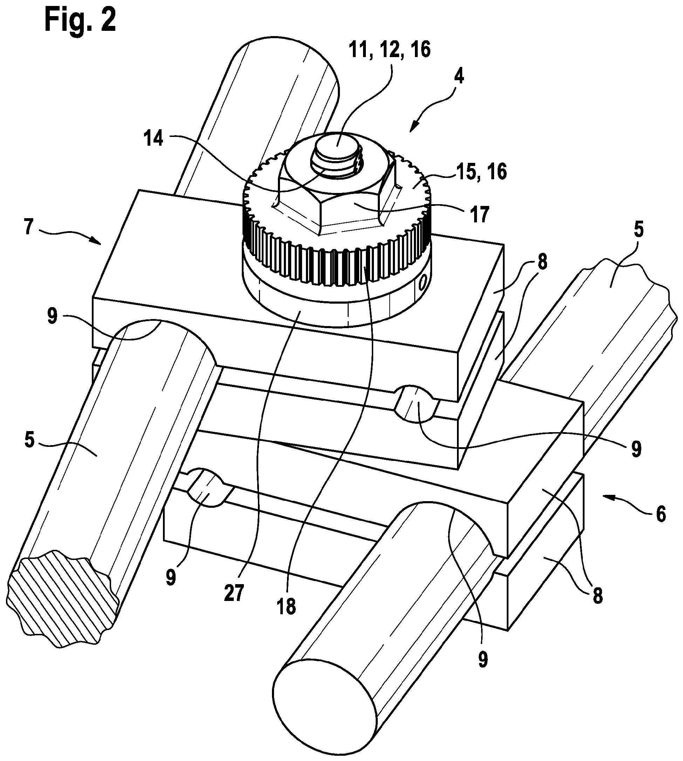

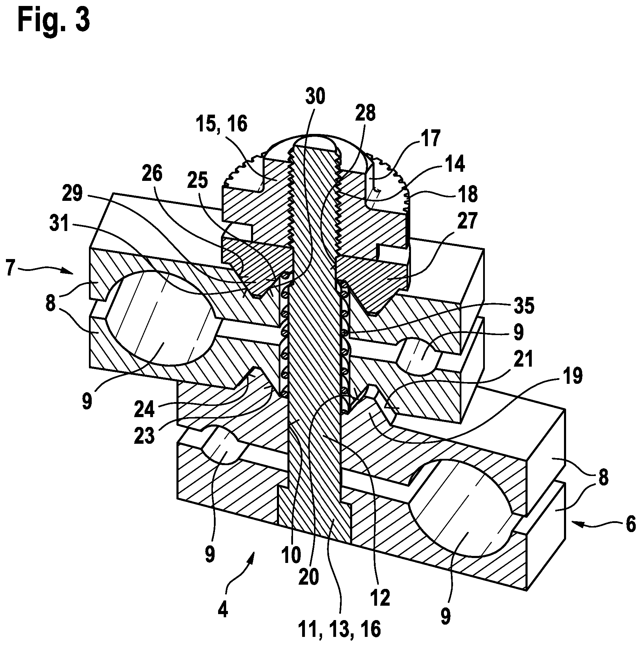

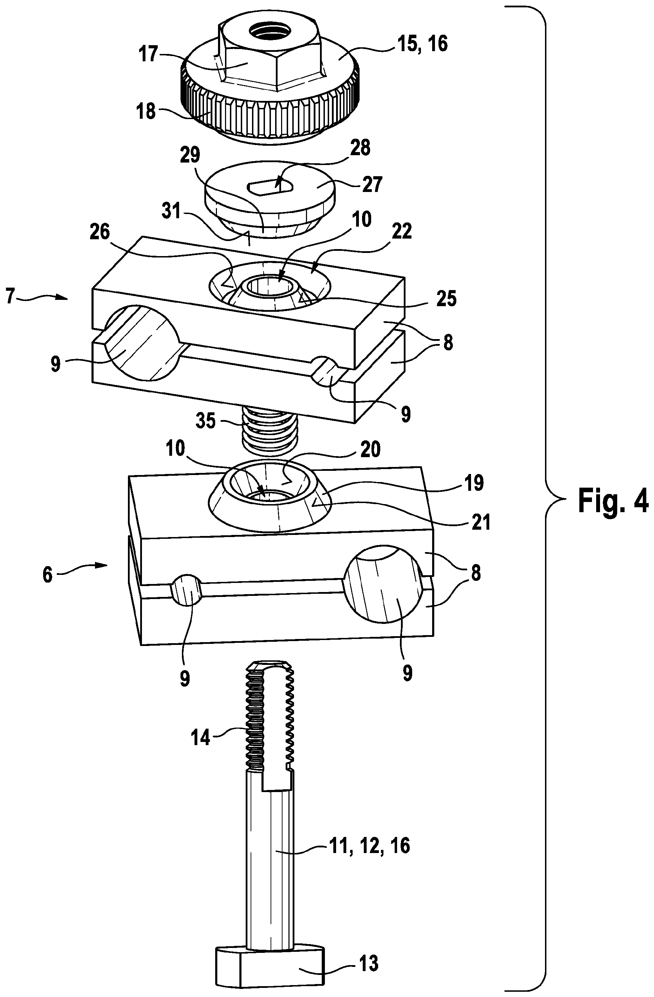

An den Pins 3 jedes Bruchstücks 2 des gebrochenen Knochens wird mithilfe von Gelenken 4 ein Stab 5 befestigt und die Stäbe 5 werden mit Gelenken 4 miteinander verbunden. Die Gelenke 4 und deren Einzelteile zeigen die Figuren 2 bis 4.

Jedes Gelenk 4 weist zwei Stabhalter 6, 7 auf, die um eine Achse gegeneinander drehbar und drehfest zusammenspannbar sind und an beziehungsweise in denen die Stäbe 5 und die Pins 3 befestigbar beziehungsweise befestigt sind. Die Stabhalter 6, 7 sind als Klemmen ausgebildet, sie weisen Klemmbacken 8 auf, zwischen denen die Pins 3 und die Stäbe 5 festklemmbar sind. Die Achse, um die die Stabhalter 6, 7 gegeneinander drehbar sind, bildet eine Achse des Gelenks 4. Jede Klemmbacke 8 kann Klemmeinrichtungen, beispielsweise Nuten, für ein, zwei oder mehr verschiedene Pins 3 und/oder Stäbe 5 aufweisen.

Die Klemmbacken 8 weisen zwei Klemmnuten 9 in einander zugewandten Innenflächen und zwischen den Klemmnuten 9 ein Durchgangsloch 10 auf. Als Innenflächen werden die einander zugewandten Seiten der Klemmbacken 8 eines Stabhalter 6, 7 bezeichnet. Die Durchgangslöcher 10 verlaufen rechtwinklig zu den Klemmnuten 9 beziehungsweise rechtwinklig zu den Innenflächen der Klemmbacken 8, in denen die Klemmnuten 9 angebracht sind. Die Durchgangslöcher 10 aller Klemmbacken 8 eines Gelenks 4 fluchten miteinander. Jeweils eine Klemmnut 9 ist so ausgeführt, dass ein Pin 3 in sie eingelegt und in ihr festgeklemmt werden kann. Die jeweils andere Klemmnut 9 ist zum Einlegen und Festklemmen eines Stabs 5 vorgesehen. Im Ausführungsbeispiel weisen die Klemmnuten 9 halbkreisförmige Querschnitte mit dem Durchmesser der Pins 3 beziehungsweise der Stäbe 5 auf. Die Klemmnuten 9 können auch andere Querschnitte, beispielsweise V- oder trapezförmige Querschnitte aufweisen (nicht dargestellt).

Durch die Durchgangslöcher 10 der Klemmbacken 8, die jeweils paarweise die beiden Stabhalter 6, 7 bilden, ist eine Schraube 11 durchgesteckt, deren Schaft eine Welle 12 bildet, die die Klemmbacken 8 beziehungsweise die Stabhalter 6, 7 drehbar um eine Längsachse der Welle 12 drehbar verbindet. Die Längsachse der Welle 12 bildet eine Achse des Gelenks 4.

Ein Schraubenkopf 13 der Schraube 11 weist zwei Parallelflächen an seinem Umfang auf und liegt in einer komplementären Senkung in einer Außenseite einer äußeren Klemmbacke 8 eines ersten der beiden Stabhalter 6 ein, in der der Schraubenkopf 13 drehfest und zugfest aufgenommen ist, so dass der Schaft der Schraube, der die Welle 12 bildet, drehfest mit der äußeren Klemmbacke 8 des ersten Stabhalters 6 verbunden ist. Schraubenköpfe 13 mit anderen Formen, beispielsweise 4- oder 6-eckige Schraubenköpfe, die in einer komplementären Senkung in der Außenseite der äußeren Klemmbacke 8 drehfest einliegen, sind ebenfalls möglich (nicht dargestellt). Die Welle 12 kann auch in anderer Weise mit der äußeren Klemmbacke 8 verbunden, beispielsweise mit ihr verschweißt sein (nicht dargestellt).

Auf ein Gewinde 14 des die Welle 12 bildenden Schafts der Schraube 11 ist eine Mutter 15 geschraubt, die die Stabhalter 6, 7 auf der Welle 12 hält. Die Schraube 11 und die Mutter 15 bilden eine Spanneinrichtung 16, mit der die Stabhalter 6, 7 beziehungsweise die Klemmbacken 8 zusammen spannbar sind. Im Ausführungsbeispiel weist die Mutter 15 einen Sechskant 17 zum Ansetzen eines nicht dargestellten Werkzeugschlüssels wie beispielsweise eines Gabelschlüssels oder eines Ringschlüssels und einen Rändel 18 an einem Umfang der Mutter 15 zum Drehen der Mutter 15 von Hand auf.

Eine innere Klemmbacke 8 des ersten Stabhalters 6 weist auf einer Außenseite eine kreisförmige Rippe 19 mit einem V-förmigen Querschnitt auf, die das Durchgangsloch 10 konzentrisch umschließt. Als innere Klemmbacke 8 wird die Klemmbacke 8 des ersten Stabhalters 6 bezeichnet, die dem anderen, also dem zweiten Stabhalter 7 des Gelenks 4 zugewandt ist. Die Außenseite ist ebenfalls dem anderen, also dem zweiten Stabhalter 7 zugewandt. Flanken der Rippe 19 bilden zwei kegelstumpfförmige Reibflächen 20, 21, wobei eine innere, innenkegelstumpfförmige Reibfläche 20 nachfolgend auch als erste Reibfläche 20 und eine äußere Reibfläche 21, die die Form eines Außenkegelstumpfs aufweist, nachfolgend auch als zweite Reibfläche 21 bezeichnet wird. Genau genommen weisen die Reibflächen 20, 21 und auch alle weiteren, noch zu beschreibenden Reibflächen 23, 24, 25, 26, 30, 31 die Form von Mantelflächen von Innen- oder Außenkegelstümpfen auf.

Beide Klemmbacken 8 des zweiten Stabhalters 7 weisen in ihren einander abgewandten Außenseiten kreisförmige Nuten 22 auf, die die Durchgangslöcher 10 der Klemmbacken 8 konzentrisch umschließen. Die Nuten 22 weisen gleiche Durchmesser wie die Rippe 19 und zu der Rippe 19 gegengleiche V-förmige Querschnitte auf. Flanken der Nuten 22 bilden zu den Reibflächen 20, 21 der Rippe 19 gegengleiche Reibflächen 23, 24, 25, 26, die nachfolgend auch als dritte, vierte, fünfte und sechste Reibfläche 23, 24, 25, 26 bezeichnet werden.

Zwischen einer äußeren Klemmbacke 8 des zweiten Stabhalter 7 und der Mutter 15 ist eine kreisförmige Scheibe 27 mit einem Mittelloch 28 auf dem die Welle 12 bildenden Schaft der Schraube 11 angeordnet. Das Mittelloch 28 weist zwei zueinander parallele Flächen auf, durch die die Scheibe 27 drehfest auf der Welle 12 ist, die im Bereich des Gewindes 14 ebenfalls zwei zueinander parallele Flächen in gleichem Abstand wie die parallelen Flächen des Mittellochs 28 der Scheibe 27 aufweist. Weil die Welle 12 drehfest mit der äußeren Klemmbacke 8 des ersten Stabhalters 6 ist, ist auch die Scheibe 27 drehfest mit der äußeren Klemmbacke 8 des ersten Stabhalters 6. Axial ist die Scheibe 27 auf der Welle 12 beweglich.

Auf ihrer der äußeren Klemmbacke 8 des zweiten Stabhalters 7 zugewandten Seite weist die Scheibe 27 eine kreisförmige Rippe 29 auf, die das Mittelloch 28 konzentrisch umschließt, die den gleichen Durchmesser wie die Nut 22 in der Klemmbacke 8 und einen gegengleichen V-förmigen Querschnitt aufweist. Die Flanken der Rippe 29 bilden eine siebte und eine achte Reibfläche 30, 31 des Gelenks 4.

Durch Festziehen der Mutter 15 auf der Schraube 11, die zusammen mit der Schraube 11 die Spanneinrichtung 16 bildet, lassen sich die Stabhalter 6, 7, die Klemmbacken 8, die Scheibe 27 und die insgesamt acht Reibflächen 20, 21, 23, 24, 25, 26, 30, 31 zusammen spannen, so dass die in den Klemmnuten 9 der Klemmbacken 8 einliegenden Stäbe 5 und Pins 3 fest zwischen den Klemmbacken 8 eingespannt und die Klemmbacken 8 beziehungsweise Stabhalter 6, 7 durch Reibung zwischen den an einander anliegenden und gegeneinander gespannten Reibflächen 20, 21, 23, 24, 25, 26, 30, 31 drehfest aneinandergehalten sind. Die Kegelform der Reibflächen 20, 21, 23, 24, 25, 26, 30, 31 bewirkt aufgrund einer Keilwirkung eine hohe Reibkraft zwischen den Reibflächen 20, 21, 23, 24, 25, 26, 30, 31 und die insgesamt acht Reibflächen 20, 21, 23, 24, 25, 26, 30, 31 verachtfachen die Reibkraft, so dass mit einer vergleichsweise niedrigen Spannkraft, die aufgebracht wird, die Stabhalter 6, 7 zuverlässig gegen Drehen aneinandergehalten sind. Es ist vorgesehen, die Mutter 15 mit einem Werkzeugschlüssel, beispielsweise einem Gabel- oder Ringschlüssel, festzuziehen. Eventuell genügt sogar ein anziehen der Mutter 15 von Hand. Denkbar ist auch, einen Schnellspanner wie er von Fahrrädern bekannt ist, oder einen anderen Exzenterhebel zum Festspannen der Spanneinrichtung 16 vorzusehen (nicht dargestellt). Ein solcher Schnellspanner wird insbesondere am Schraubenkopf 13 vorgesehen.

Durch Lösen der Mutter 15 lassen sich die Stäbe 5 und Pins 3 in den Klemmnuten 9 der Klemmbacken 8 verschieben und die Klemmbacken 8 beziehungsweise die Stabhalter 6, 7 gegeneinander verdrehen, wodurch sich die Stäbe 5 und die Pins 3 und die an ihnen befestigten Bruchstücke 2 des Knochens oder Skelettteile in eine gewünschte Lage zueinander bringen lassen. Zum Ausrichten der Stabhalter 6, 7, Klemmbacken 8, Stäbe 5 und Pins 3 lässt sich die Reibkraft zwischen den Reibflächen 20, 21, 23, 24, 25, 26, 30, 31 durch die mit der Mutter 15 erzeugte Spannkraft fein dosiert einstellen.

Spitzen der Rippen 19, 29 sind abgeflacht, damit sie nicht auf einem Grund der Nuten 22 aufsitzen.

In zylindrischen Senkungen der Durchgangslöcher 10 der inneren Klemmbacken 8 der beiden Stabhalter 6, 7 ist eine Schraubendruckfeder als Abdrückfeder 35 angeordnet, die die Klemmbacken 8 und die Reibflächen 20, 21, 23, 24, 25, 26, 30, 31 voneinander abdrückt, wenn sie nicht mit der Spanneinrichtung 16 zusammen gespannt werden. Dadurch lassen sich die Reibkräfte zwischen den Reibflächen 20, 21, 23, 24, 25, 26, 30, 31 zum Schwenken der Stäbe 5 und der Pins 3 gut mit der Mutter 15 dosieren.

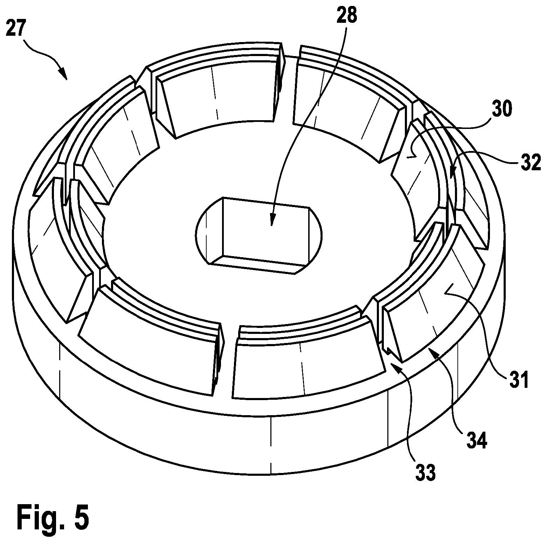

Figur 5 zeigt eine Scheibe 27 mit einer alternativ ausgeführten Rippe 29 mit V-förmigem Querschnitt, deren Flanken die siebte und die achte Reibfläche 30, 31 bilden. Die Rippe 29 ist durch einen zylinderförmigen Schlitz 32 in einen äußeren und einen inneren Teil geteilt. Außerdem teilen radiale Schlitze 33 die Rippe 29 in mehrere Sektoren. Außerdem ist ein Schlitz 34 in einer Radialebene an einem Fuß der Rippe 29 angebracht, im Ausführungsbeispiel nur von außen. Durch die Schlitze 32, 33, 34 können die Reibflächen 30, 31 in radialer Richtung federn, sie sind radialelastisch. Dadurch können sich die Reibflächen 30, 31 zum Ausgleich von Fertigungstoleranzen an die gegengleichen Reibflächen 23, 24, 25, 26 in den Nuten in den Außenseiten der Klemmbacken 8 des zweiten Stabhalters 7 anpassen. Auch die Rippe 19 auf der Außenseite der inneren Klemmbacke 8 des ersten Stabhalters 6 kann solche Schlitze 32, 33, 34 aufweisen.

## Patentansprüche

1. Äußere Haltevorrichtung zum Halten von Bruchstücken eines gebrochenen Knochens oder allgemein von Skelettteilen, mit zwei Stäben (5) und einem Gelenk (4), das die beiden Stäbe (5) um eine Achse drehbar verbindet und das gegen Drehung feststellbar ist, wobei das Gelenk (4) zwei Stabhalter (6, 7), an denen die Stäbe (5) befestigt sind, aufweist und wobei die beiden Stabhalter (6, 7) um die Achse gegeneinander drehbar sind, **dadurch gekennzeichnet, dass** ein erster der beiden Stabhalter (6) eine erste, kegelstumpfförmige und zu der Achse konzentrische Reibfläche (20) und der zweite Stabhalter (7) eine gegengleiche ebenso kegelstumpfförmige, der ersten Reibfläche (20) zugewandte und zu der Achse konzentrische dritte Reibfläche (23) aufweist, und dass die Haltevorrichtung (1) eine Spanneinrichtung (16) aufweist, mit der die beiden Reibflächen (20, 23) gegeneinander spannbar sind, so dass die beiden Stabhalter (6, 7) durch Reibschluss zwischen den kegelstumpfförmigen Reibflächen (20, 23) gegen Drehen aneinander gehalten sind.

2. Haltevorrichtung nach Anspruch 1, **dadurch gekennzeichnet, dass** der erste Stabhalter (6) eine kegelstumpfförmige, zu der Achse konzentrische zweite Reibfläche (21), die die erste Reibfläche (20) konzentrisch umschließt, und der zweite Stabhalter (7) eine zu der zweiten Reibfläche (21) gegengleiche, kegelstumpfförmige, der zweiten Reibfläche (21) zugewandte, zu der Achse konzentrische vierte Reibfläche (24) aufweist, die die dritte Reibfläche (23) konzentrisch umschließt, und dass die Spanneinrichtung (16) zugleich mit der ersten und der dritten Reibfläche (20, 23) auch die zweite und die vierte Reibfläche (21, 24) gegeneinander spannt, so dass zusätzlich zu der ersten und der dritten Reibfläche (20, 23) auch die zweite und die vierte Reibfläche (21, 24) durch Reibschluss zwischen sich die beiden Stabhalter (6, 7) gegen Drehen halten.

3. Haltevorrichtung nach Anspruch 1 oder 2, **dadurch gekennzeichnet, dass** der zweite Stabhalter (7) eine fünfte, kegelstumpfförmige, zu der Achse konzentrische Reibfläche (25) aufweist, die dem ersten Stabhalter (6) abgewandt ist, und dass das Gelenk (4) eine kegelstumpfförmige, zu der Achse konzentrische, zu der fünften Reibfläche (25) gegengleiche und der fünften Reibfläche (25) zugewandte siebte Reibfläche (30) aufweist, die der fünften Reibfläche (25) zugewandt und die drehfest mit dem ersten Stabhalter (6) ist, und dass die Spanneinrichtung (16) zugleich mit der ersten und der dritten Reibfläche (20, 23) auch die fünfte und die siebte Reibfläche (25, 30) gegeneinander spannt, so dass zusätzlich zu der ersten und der dritten Reibfläche (20, 23) auch die fünfte und die siebte Reibfläche (25, 30) durch Reibschluss zwischen sich die beiden Stabhalter (6, 7) gegen Drehen halten.

4. Haltevorrichtung nach Anspruch 3, **dadurch gekennzeichnet, dass** der zweite Stabhalter (7) eine sechste, kegelstumpfförmige, zu der Achse konzentrische Reibfläche (26) aufweist, die dem ersten Stabhalter (6) abgewandt ist und die die fünfte Reibfläche (25) konzentrisch umschließt, und dass das Gelenk (4) eine kegelstumpfförmige, zu der Achse konzentrische, zu der sechsten Reibfläche (26) gegengleiche und der sechsten Reibfläche (26) zugewandte achte Reibfläche (31) aufweist, die drehfest mit dem ersten Stabhalter (6) ist, und dass die Spanneinrichtung (16) zugleich mit der ersten und der dritten Reibfläche (20, 23) auch die sechste und die achte Reibfläche (26, 31) gegeneinander spannt, so dass zusätzlich zu den anderen Reibflächen (20, 21, 23, 24, 25, 30) auch die sechste und die achte Reibfläche (26, 31) durch Reibschluss zwischen sich die beiden Stabhalter (6, 7) gegen Drehen halten.

5. Haltevorrichtung nach Anspruch 3 oder 4, **dadurch gekennzeichnet, dass** das Gelenk (4) eine mit dem ersten Stabhalter (6) drehfeste, zu der Achse koaxiale Welle (12) aufweist, die den ersten Stabhalter (6) durchgreift und die siebte Reibfläche (30) drehfest mit dem ersten Stabhalter (6) verbindet.

6. Haltevorrichtung nach Anspruch 5, **dadurch gekennzeichnet, dass** die Welle (12) Teil der Spanneinrichtung (16) ist und dass die Welle (12) eine axiale Spannkraft zum gegeneinander Spannen der Reibflächen (20, 21, 23, 24) von dem zweiten Stabhalter (7) auf die siebte Reibfläche (30) überträgt.

7. Haltevorrichtung nach einem oder mehreren der vorhergehenden Ansprüche, **dadurch gekennzeichnet, dass** das Gelenk (4) eine Abdrückfeder (35) aufweist, die die Reibflächen (20, 21, 23, 24, 25, 26, 30, 31) voneinander abdrückt, wenn sie nicht von der Spanneinrichtung (16) gegeneinander gespannt werden, so dass sich die beiden Stabhalter (6, 7) mit den beiden Stäben (5) einstellbar leichtgängig gegeneinander drehen lassen, wenn die Spanneinrichtung (16) gelöst ist.

8. Haltevorrichtung nach einem oder mehreren der vorhergehenden Ansprüche, **dadurch gekennzeichnet, dass** Reibflächen (20, 21, 23, 24, 25, 26, 30, 31) radialelastisch sind.

9. Haltevorrichtung nach einem oder mehreren der vorhergehenden Ansprüche, **dadurch gekennzeichnet, dass** die beiden Stabhalter (6, 7) lösbare Klemmen aufweist, in denen die beiden Stäbe (2, 3) festgeklemmt sind.

10. Haltevorrichtung nach Anspruch 9, **dadurch gekennzeichnet, dass** mindestens ein Stabhalter (6, 7) eine Klemme mit Klemmeinrichtungen für Stäbe (2, 3) mit unterschiedlichen Stabquerschnitten aufweist.
